# FASCICULE DE BREVET EUROPEEN

(11) **EP 1 607 069 B1**
(45) Date de publication et mention de la délivrance du brevet: **23.12.2009**
(21) Numéro de dépôt: 05356105.6
(22) Date de dépôt: 14.06.2005
(51) Int. Cl.: A61F 2/40

(54) **Prothese totale d'epaule de type inverse**
Inverse Schultertotalprothese
Inverse-type total shoulder prosthesis

(30) Priorité: 15.06.2004 FR 0406472
(43) Date de publication de la demande: 21.12.2005
(73) Titulaire: TORNIER, 38330 Saint-Ismier (FR)
(72) Inventeur: Tornier, Alain, 38330 Saint Ismier (FR); Walsch, Gilles, 69003 Lyon (FR); Boileau, Pascal, 06200 Nice (FR)
(74) Mandataire: Myon, Gérard Jean-Pierre

(56) Documents cités:
- EP-A- 0 299 889
- FR-A- 2 652 498
- FR-A- 2 699 400

## Description

L'invention a trait à une prothèse d'épaule et, plus spécifiquement à une prothèse d'épaule dont la compacité peut être améliorée par rapport à celles de l'état de la technique.

Dans le domaine des prothèses d'épaule, il est connu, par exemple de EP-A-0 299 889, de constituer une prothèse dite « inversée » dans laquelle une surface articulaire convexe solidaire de la glène et une surface articulaire concave solidaire de l'humérus coopèrent pour recréer une articulation au niveau de l'épaule, avec un centre de rotation situé dans la glène. Dans ce genre de prothèses, la surface articulaire convexe est souvent réalisée en métal, alors qu'une coupelle en matériau synthétique, par exemple en polyéthylène, est conformée pour créer la surface concave d'articulation humérale. Une telle coupelle doit, pour résister aux efforts auxquels elle est soumise, présenter une épaisseur relativement importante ce qui induit un encombrement latéral de la prothèse qui peut parfois gêner le patient, notamment lors des mouvements d'abduction.

C'est à ces inconvénients qu'entend plus particulièrement remédier l'invention en proposant une nouvelle prothèse totale d'épaule de type inversé dont l'encombrement latéral risque moins de gêner le patient que les prothèses de l'état de la technique.

Dans cet esprit, l'invention concerne une prothèse totale d'épaule comprenant un composant glénoïdien, définissant une surface articulaire convexe sensiblement hemisphérique et dont le centre de courbure est situé dans la glène ou à proximité immédiate de la glène lorsque le composant glénoïdien est en place sur la glène, et un composant huméral définissant une surface articulaire concave adaptée pour coopérer avec la surface articulaire convexe du composant glénoïdien **caractérisée en ce que** la surface articulaire concave est définie par une partie métallique du composant huméral.

Grâce à l'invention, on peut optimiser l'encombrement latéral de la prothèse, tout en conservant le caractère fonctionnel de la surface articulaire humérale.

Selon des aspects avantageux mais non obligatoires, une prothèse totale d'épaule de type inversé peut incorporer une ou plusieurs des caractéristiques suivantes, prises dans toute combinaison techniquement admissible :
- La surface articulaire convexe est définie dans une partie métallique du composant glénoïdien. Il s'est avéré, de façon surprenante, qu'une prothèse de type inversé dans laquelle les deux surfaces articulaires sont réalisées en métal permet des mouvements aisés du patient, alors que le fait de se passer de polyéthylène autorise une meilleure compacité de la prothèse, notamment une diminution de son encombrement latéral.
- Les parties métalliques dans lesquelles sont réalisées les surfaces articulaires sont en titane ou en alliage à base de chrome-cobalt.
- Selon un premier mode de réalisation envisageable, le composant huméral comprend une partie métaphysaire monobloc qui définit la surface articulaire concave.
- Selon un autre mode de réalisation, le composant huméral comprend une partie métaphysaire bipartite formée d'un corps sur lequel est monté un insert métallique définissant la surface articulaire concave. Dans ce cas, l'insert est avantageusement immobilisé sur le corps par coopération de formes. On peut, en outre, prévoir que ce corps est apte à recevoir différents implants qui définissent des surfaces articulaires différentes et/ou positionnées différemment par rapport à ce corps, ce qui permet d'adapter, dans une certaine mesure, la prothèse à son site d'implantation.
- Quel que soit le mode de réalisation considéré, la partie métaphysaire du composant huméral peut être monobloc avec ou rapporté sur une queue d'ancrage de ce composant dans l'humérus.
- Le composant huméral est entièrement métallique.

L'invention sera mieux comprise et d'autres avantages de celle-ci apparaîtront plus clairement à la lumière de la description qui va suivre de deux modes de réalisation d'une prothèse conforme à son principe, donnée uniquement à titre d'exemple et faite en référence aux dessins annexés dans lesquels :
- la figure 1 est une représentation schématique de principe d'une prothèse conforme à l'invention implantée au niveau de l'épaule d'un patient ;
- la figure 2 est une vue en perspective du composant huméral de la prothèse de la figure 1 ;
- la figure 3 est une vue de côté d'une partie du composant huméral et du composant glénoïdien de la prothèse de la figure 1, en configuration séparée l'un de l'autre ;
- la figure 4 est une vue en perspective du composant huméral d'une prothèse conforme à un second mode de réalisation de l'invention, et
- la figure 5 est une vue analogue à la figure 3 pour la prothèse dont l'élément huméral est représenté à la figure 4.

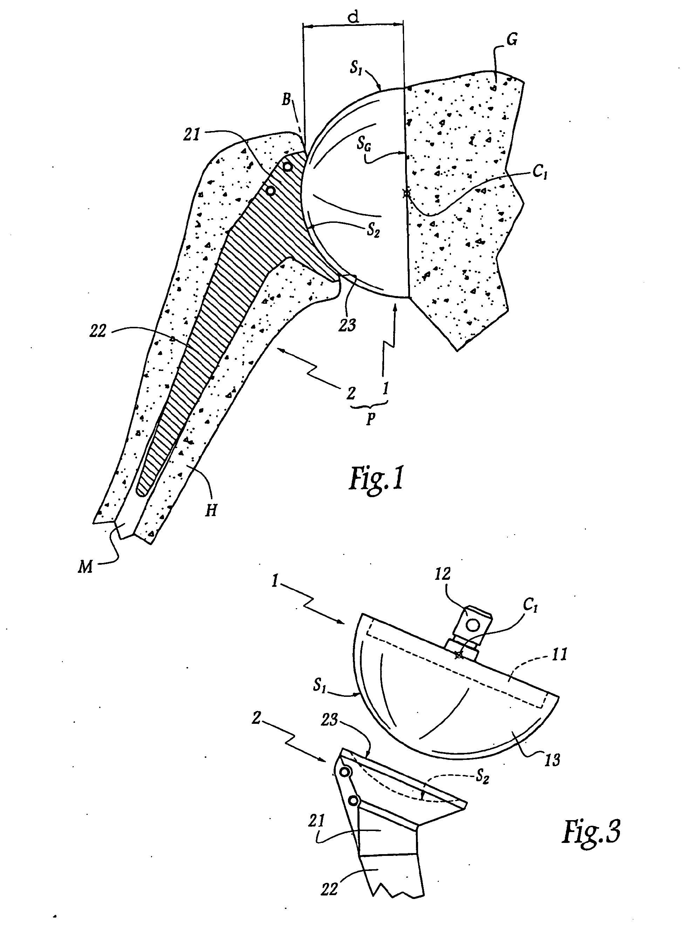

La prothèse P représentée à la figure 1 comprend un composant glénoïdien 1 qui définit une surface articulaire convexe S₁ de forme globalement hémisphérique et dont le centre de courbure C₁ est situé au niveau de la face arrière du composant 1, c'est-à-dire au niveau de la surface fraisée S_{G} de la glène. Ainsi qu'il ressort plus particulièrement de la figure 3, le composant 1 comprend une embase 11 pourvue d'une queue d'ancrage 12 et destinée à être fixée sur la glène G au moyen de plusieurs vis qui ne sont pas représentées. Une tête métallique 13 est prévue pour être montée sur l'embase 11 et conformée pour définir la surface S₁. Ce composant glénoïdien peut, par exemple, être conforme à l'enseignement technique du brevet français 2 835 425.

La prothèse P comprend également un composant huméral 2 qui est également métallique. Le composant 2 comprend une partie métaphysaire 21 monobloc avec une queue 22 d'ancrage dans le canal médulaire M de l'humérus H. La partie 21 est usinée en creux et définit une surface concave S₂ en forme de tronçon de sphère de rayon sensiblement égal à celui de la surface S₁.

La surface S₂ est bordée par un bord annulaire et circulaire 23.

Lorsque la prothèse est implantée, comme représenté à la figure 1, où le composant 2 est représenté en coupe alors que le composant 1 est représenté en vue extérieure pour la clarté du dessin, les surfaces S₁ et S₂ sont en appui surfacique l'une contre l'autre, ce qui permet les différents mouvements d'épaule recherchés.

Compte tenu du fait que la surface S₂ est formée par la partie 21 elle-même, la distance d entre la surface réséquée S_{G} de la glène et le bord coupé B de l'humérus H réséqué est relativement petite vis-à-vis d'une même distance pour une prothèse connue.

La partie 13 et le composant 2 sont avantageusement réalisés dans un métal bio-compatible, par exemple en titane ou dans un alliage à base de chrome-cobalt.

Selon une variante avantageuse de l'invention, la surface S₂ n'est pas nécessairement centrée dans la partie métaphysaire 21. Par exemple, le composant 2 peut être sélectionné dans un jeu de composants dont les surfaces S₂ sont plus ou moins excentrées par rapport à leurs parties métaphysaires respectives.

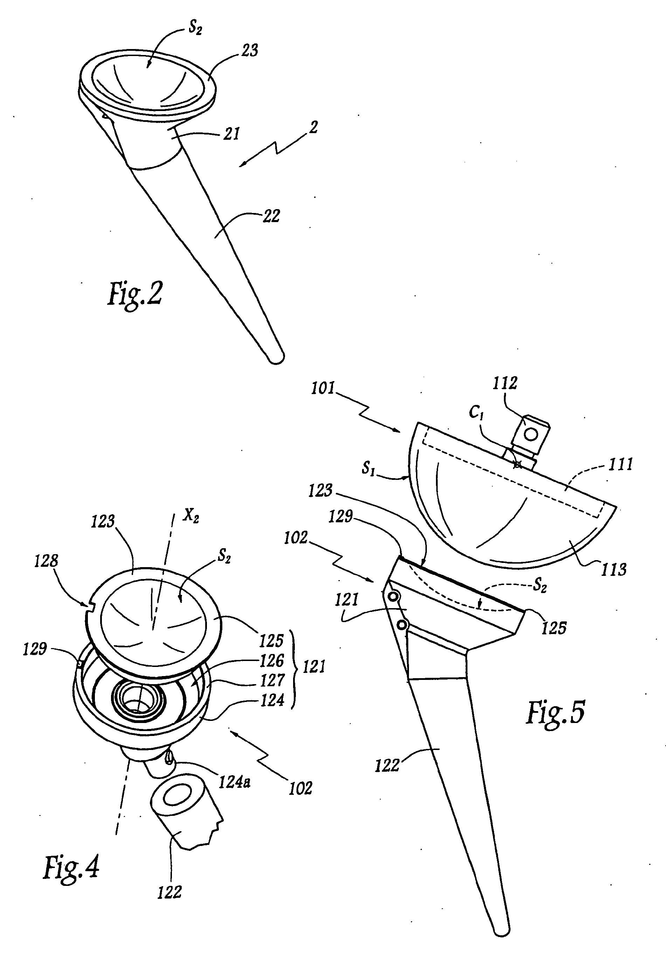

Dans le second mode de réalisation de l'invention représenté aux figures 4 et 5, les éléments analogues à ceux du premier mode de réalisation portent des références identiques augmentées de 100. Le composant glénoïdien 101 de ce mode de réalisation comprend également une embase 111 dont la queue 112 permet l'ancrage dans la glène ainsi qu'une tête métallique 113 définissant une surface articulaire hémisphérique et convexe S₁.

Le composant huméral 102 comprend une partie métaphysaire 121 constituée d'un corps 124 et d'un insert 125 métalliques. L'insert et le corps sont avantageusement réalisés dans le même métal ou alliage métallique, par exemple en titane ou en alliage à base de chrome-cobalt.

L'insert 125 est destiné à être immobilisé sur le corps 124 par coopération de formes en étant partiellement engagé dans le volume interne 126 du corps 124, un bord périphérique 123 de l'insert 125 venant en appui sur un bord périphérique 127 du corps 124.

L'insert 125 définit une surface hémisphérique concave S₂ de même géométrie que celle de la prothèse du premier mode de réalisation.

L'insert 125 est pourvu d'une encoche 128 au niveau de son bord 123, alors que le corps 124 porte un téton 129 sur son bord 127. La coopération des éléments 128 et 129 permet d'immobiliser l'insert 125 en rotation autour d'un axe X₂-X'₂ de la surface S₂.

Par ailleurs, la partie 121 est rapportée sur une queue d'ancrage 122 destinée à être immobilisée dans le canal médullaire huméral. Pour ce faire, le corps 124 est pourvu d'une extension 124a destinée à être introduite et coincée à l'intérieur d'un logement 122a de forme correspondante prévue dans la queue 122.

Comme précédemment, le fait de réaliser les surfaces S₁ et S₂ dans les parties 113 et 125 qui sont métalliques permet d'améliorer la compacité de la prothèse, tout en autorisant des mouvements aisés et de grande amplitude du bras du patient par rapport à la glène.

Selon un aspect non représenté de l'invention, on peut prévoir que des inserts 125 de formes différentes peuvent être montés sur le corps 124, ce qui permet d'adapter la prothèse à son site d'implantation. Par exemple, des inserts 125 dont les surfaces S₂ ont des rayons de courbures différents peuvent être utilisés, l'un d'entre eux étant sélectionné et monté sur le corps 124 en fonction de la géométrie de la surface S₁ utilisée pour le composant glénoïdien. De même, des inserts dans lesquels les surfaces S₂ sont positionnées différemment par rapport au bord 123 peuvent être utilisés, l'un d'entre eux étant sélectionné lors de la pose de la prothèse en fonction de la morphologie du patient.

Les caractéristiques des modes de réalisation décrits peuvent être combinées entre elles dans le cadre de la présente invention. En particulier, une partie métaphysaire monobloc, telle que celle du premier mode de réalisation, peut être rapportée sur une queue d'ancrage, telle que celle du second mode de réalisation. De même, le corps du second mode de réalisation, peut être monobloc avec une queue d'ancrage, comme dans le premier mode de réalisation.

## Revendications

1. Prothèse totale d'épaule comprenant un composant glénoïdien (1 ; 101), définissant une surface articulaire convexe (S₁) sensiblement hémisphérique et dont le centre de courbure (C₁) est situé dans la glène (G) ou au voisinage immédiat (S_{G}) de celle-ci lorsque le composant glénoïdien est en place sur la glène, et un composant huméral (2 ; 102) définissant une surface articulaire concave (S₂) adaptée pour coopérer avec la surface articulaire convexe dudit composant glénoïdien, **caractérisée en ce que** ladite surface articulaire concave (S₂) est définie par une partie métallique (21 ; 125) dudit composant huméral (2 ; 102).

2. Prothèse selon la revendication 1, **caractérisée en ce que** ladite surface articulaire convexe (S₁) est définie par une partie métallique (13 ; 113) dudit composant glénoïdien (1 ; 101).

3. Prothèse selon l'une des revendications précédentes, **caractérisée en ce que** ladite ou lesdites parties métalliques (13, 21 ; 113, 125) sont en titane ou en alliage à base de chrome-cobalt.

4. Prothèse selon l'une des revendications précédentes, **caractérisée en ce que** ledit composant huméral (2) comprend une partie métaphysaire monobloc (21) qui définit ladite surface articulaire concave (S₂).

5. Prothèse selon l'une des revendications 1 à 3, **caractérisée en ce que** ledit composant huméral (102) comprend une partie métaphysaire bipartite (121) formée d'un corps (124) sur lequel est monté un insert métallique (125) définissant ladite surface articulaire concave (S₂).

6. Prothèse selon la revendication 5, **caractérisée en ce que** ledit insert (125) est immobilisé sur ledit corps (124) par coopération de formes.

7. Prothèse selon l'une des revendications 5 ou 6, **caractérisée en ce que** ledit corps (124) est apte à recevoir différents implants (125) qui définissent des surfaces articulaires (S₂) différentes et/ou positionnées différemment les unes des autres par rapport audit corps.

8. Prothèse selon l'une des revendications 4 à 7, **caractérisée en ce que** ladite partie métaphysaire (21) est monobloc avec une queue (22) d'ancrage dudit composant huméral dans l'humérus (H).

9. Prothèse selon l'une des revendications 4 à 7, **caractérisée en ce que** ladite partie métaphysaire (121) est rapportée sur une queue (122) d'ancrage dudit composant huméral dans l'humérus (H).

10. Prothèse selon l'une des revendications précédentes, **caractérisée en ce que** ledit composant huméral (2 ; 102) est entièrement métallique.

## Claims

1. Total shoulder prosthesis comprising a glenoidal component (1 ; 101), defining a substantially hemispherical convex articular surface (S₁) of which the centre of curvature (C₁) is located in the glene (G) or in the immediate vicinity (S₄) thereof when the glenoidal component is installed on the glene, and a humeral component (2 ; 102) defining a concave articular surface (S₂) adapted to cooperate with the convex articular surface of said glenoidal component,
wherein said concave articular surface (S₂) is defined by a metallic part (21 ; 125) of said humeral component (2 ; 102).

2. The prosthesis of Claim 1, wherein said convex articular surface (S₁) is defined by a metallic part (13 ; 113) of said glenoidal component (1 ; 101).

3. The prosthesis of one of the previous claims, wherein said metallic part(s) (13 ; 21 ; 113, 125) are made of titanium or a chromium-cobalt alloy.

4. The prosthesis of one of the previous claims, wherein said humeral component (2) comprises a one-piece metaphyseal part (21) which defines said concave articular surface (S₂).

5. The prosthesis of one of the previous claims, wherein said humeral component (102) comprises a two-piece metaphyseal part (121) formed by a body (124) on which is mounted a metallic insert (125) defining said concave articular surface (S₂).

6. The prosthesis of Claim 5, wherein said insert (125) is immobilized on said body (124) by cooperation of shapes.

7. The prosthesis of Claim 5 or 6, wherein said body (124) is adapted to receive different implants (125) which define articular surfaces (S₂) which are different and/or positioned differently with respect to said body.

8. The prosthesis of one of Claims 4 to 7, wherein said metaphyseal part (21) is in one piece with a stem (22) for anchorage of said humeral component in the humerus (H).

9. The prosthesis of one of Claims 4 to 7, wherein said metaphyseal part (121) is added on a stem (122) for anchorage of said humeral component in the humerus (H).

10. The prosthesis of one of the previous claims, wherein said humeral component (2 ; 102) is entirely metallic.

## Patentansprüche

1. Schultertotalprothese, umfassend eine Glenoidkomponente (1; 101), die eine im Wesentlichen halbkugelförmige, konvexe Gelenkoberfläche (S₁) definiert, deren Krümmungszentrum (C₁) sich in der Gelenkpfanne (G) oder in unmittelbarerer Umgebung (S_{G}) von dieser befindet, wenn sich die Glenoidkomponente in der Gelenkpfanne befindet, sowie eine Humeruskomponente (2; 102), die eine konkave Gelenkoberfläche (S₂) definiert, die derart gestaltet ist, dass sie mit der konvexen Gelenkoberfläche der Glenoidkomponente zusammenwirkt, **dadurch gekennzeichnet, dass** die konkave Gelenkoberfläche (S₂) durch einen metallischen Abschnitt (21; 125) der Humeruskomponente (2; 102) definiert ist.

2. Prothese nach Anspruch 1, **dadurch gekennzeichnet, dass** die konvexe Gelenkoberfläche (S₁) durch einen metallischen Abschnitt (13; 113) der Glenoidkomponente (1; 101) definiert ist.

3. Prothese nach einem der vorhergehenden Ansprüche, **dadurch gekennzeichnet, dass** der oder die metallische(n) Abschnitt(e) (13, 21; 113, 125) aus Titan oder aus einer Legierung auf Chrom-Kobalt-Basis sind.

4. Prothese nach einem der vorhergehenden Ansprüche, **dadurch gekennzeichnet, dass** die Humeruskomponente (2) einen einteiligen Metaphysenabschnitt (21) umfasst, der die konkave Gelenkoberfläche (S₂) definiert.

5. Prothese nach einem der Ansprüche 1 bis 3, **dadurch gekennzeichnet, dass** die Humeruskomponente (102) einen zweiteiligen Metaphysenabschnitt (121) umfasst, der gebildet wird aus einem Körper (124), auf den ein metallischer Einsatz (125) aufgebracht ist, der die konkave Gelenkoberfläche (S₂) definiert.

6. Prothese nach Anspruch 5, **dadurch gekennzeichnet, dass** der Einsatz (125) auf dem Körper (124) mittels formschlüssiger Verbindung immobilisiert ist.

7. Prothese nach einem der Ansprüche 5 oder 6, **dadurch gekennzeichnet, dass** der Körper (124) dafür ausgelegt ist, verschiedene Implantate (125) aufzunehmen, die Gelenkoberflächen (S₂) definieren, die verschieden sind oder zueinander in Bezug auf den Körper unterschiedlich positioniert sind.

8. Prothese nach einem der Ansprüche 4 bis 7, **dadurch gekennzeichnet, dass** der Metaphysenabschnitt (21) einen Monoblock mit einem Verankerungsstiel (22) für die Verankerung der Humeruskomponente im Humerus (H) bildet.

9. Prothese nach einem der Ansprüche 4 bis 7, **dadurch gekennzeichnet, dass** der Metaphysenabschnitt (121) auf einem Verankerungsstiel (122) für die Verankerung der Humeruskomponente im Humerus (H) angebracht ist.

10. Prothese nach einem der vorhergehenden Ansprüche, **dadurch gekennzeichnet, dass** die Humeruskomponente (2; 102) vollständig aus Metall ist.
